# EUROPÄISCHE PATENTSCHRIFT

(11) **EP 0 726 320 B1**
(45) Veröffentlichungstag und Bekanntmachung des Hinweises auf die Patenterteilung: **11.08.1999**
(21) Anmeldenummer: 96101776.1
(22) Anmeldetag: 07.02.1996
(51) Int. Cl.: C12N 15/69, C12N 15/53, C12N 1/21

(54) **Mikrobielle Herstellung von 5-Ketogluconat**
Microbiological preparation of 5-cetogluconate
Préparation microbiologique de 5-cétogluconate

(30) Priorität: 07.02.1995 DE 19503946
(43) Veröffentlichungstag der Anmeldung: 14.08.1996
(73) Patentinhaber: RHEIN BIOTECH GESELLSCHAFT FUR NEUE BIOTECHNOLOGISCHE PROZESSE UND PRODUKTE MBH, 40595 Düsseldorf (DE)
(72) Erfinder: Klasen, Ralf, D-51373 Leverkusen (DE); Bringer-Meyer, Stephanie, D-52428 Jülich (DE); Sahm, Herman, D-52428 Jülich (DE); Hollenberg, Cornelis Petrus, 40479 Düsseldorf (DE)
(74) Vertreter: Grünecker, Kinkeldey, Stockmair & Schwanhäusser Anwaltssozietät

(56) Entgegenhaltungen:
- JOURNAL OF BACTERIOLOGY, Bd. 177, Nr. 10, 1995, Seiten 2637-2643, XP002010199 R. KLASEN ET AL.: "Biochemical characterization and sequence analysis of the gluconate:NADP 5-oxidoreductase gene fron Gluconobacter oxydans"
- AGRIC. BIOL. CHEM., Bd. 43, Nr. 1, 1979, Seiten 75-83, XP002010200 O. ADACHI ET AL.: "Crystallisation and properties of 5-keto-D-gluconate reductase from Gluconbacter suboxydans"

## Beschreibung

Die Erfindung betrifft ein Gluconat:NADP⁺-5-Oxidoreduktase-Gen, transformierte Zellen, die dieses Gen enthalten, sowie ein Verfahren zur mikrobiellen Herstellung von 5-Ketogluconat.

Es ist bekannt, daß Bakterien, wie beispielsweise Gluconobacter, Glucose oxidativ zu Gluconat und Gluconat weiter zu 5-Ketogluconat umsetzen, das anschließend aus dem Kulturmedium gewinnbar ist.

Die mikrobielle Herstellung und Gewinnung von 5-Ketogluconat ist für die Ascorbinsäure- und insbesondere für die L-(+)-Weinsäureherstellung von Interesse. Derzeit wird die L-(+)-Weinsäure aus Weinstein, einem bei der Weinherstellung anfallenden Nebenprodukt, gewonnen. Damit ist die Weinsäureproduktion von der Verfügbarkeit dieses Rohstoffes abhängig. Auch ist der Rohweinstein in der Regel durch organische Materialien verunreinigt, so daß es zur Gewinnung der Weinsäure aufwendiger Aufarbeitungen bedarf.

Aufgrund dieser Nachteile wird versucht, alternative Verfahren zur Herstellung der Weinsäure zu entwickeln. Eine alternative Verfahrensweise bildet insbesondere die Umsetzung von mikrobiell gewonnenem 5-Ketogluconat zu L-(+)-Weinsäure (vgl. auch DE-Patentanmeldung Nr. P 44 40 191.4 - 44). Für einen wirtschaftlichen Einsatz dieses Verfahrens sind aber die mikrobiell erreichten 5-Ketogluconatausbeuten derzeit noch zu gering:

So bildet beispielsweise Gluconobacter oxydans bei Wachstum auf Glucose stets 2- und 5-Ketogluconat, wobei stammspezifisch unterschiedliche Ausbeuten erhalten werden: Für die 2-Ketogluconatbildung werden Ausbeuten im Bereich von 4% bis 97% und für die 5-Ketogluconatbildung Ausbeuten im Bereich von 4% bis 35% - jeweils bezogen auf die umgesetzte Glucose - beschrieben (Weenk, G., Olijve, W. und Harder, W., 1984, Ketogluconate formation by Gluconobacter species. Appl. Microbiol. Biotechnol. 20, 400-405). Durch unterschiedliche Kulturbedingungen wird die bevorzugte Bildung jeweils einer der beiden Ketogluconate erreicht: So führt ein pH-Wert von 4, eine Inkubationstemperatur von 25°C und die Titration mit CaCO₃ zu einer verstärkten 5-Ketogluconatbildung (Stadler-Szöke, A., Nyeste, L. und Hollo, J., 1980, Studies on the factors effecting gluconic acid und 5-ketogluconic acid formation by Acetobacter. Acta Aliment. 9, 155-172). Höhere pH-Werte und höhere Temperaturen fördern dagegen die 2-Ketogluconatbildung (Ameyama, M. und Kondo, K., 1958, Carbohydrate metabolism by Acetobacter species. Bull. Agric. Chem. Soc. Japan 22, 373-379).

Es ist daher Aufgabe der Erfindung, Mittel bereitzustellen, die es erlauben, 5-Ketogluconat in ausreichenden Mengen auf unkomplizierte Weise zu erzeugen, so daß die mikrobielle Herstellung wirtschaftlich rentabel wird.

Diese Aufgabe wird erfindungsgemäß dadurch gelöst, daß ein Gluconat: NADP⁺-5-Oxidoreduktase-Gen nach Anspruch 1 zur Verfügung gestellt wird.

Es wurden Gluconat-Oxidoreduktase-Gene isoliert und sequenziert, die für die in Tabelle 1 angegebene Aminosäuresequenz oder deren Allelvariationen kodieren. Allelvariationen umfassen insbesondere funktionelle Derivate, die durch Deletion(en), Insertion(en) oder Substitution(en) von Nukleotiden aus entsprechenden Sequenzen erhältlich sind, wobei die Gluconat-Oxidoreduktase-Aktivität aber erhalten bleibt. Eine entsprechende Sequenz ist in Tabelle 1 angegeben.

Dem Gluconat-Oxidoreduktase-Gen sind vorzugsweise regulatorische Gensequenzen zugeordnet, die insbesondere die Genaktivität erhöhen. So kann durch Mutation der regulatorischen Gensequenz die Effektivität der Bindung des Regulatorproteins an die DNA des zu regulierenden Gluconat-Oxidoreduktase-Gens so beeinflußt sein, daß dadurch die Transkription verstärkt und somit die Genepression erhöht ist. Desweiteren können dem Gluconat-Oxidoreduktase-Gen als regulatorische Sequenzen auch sog. "enhancer" zugeordnet sein, die über eine verbesserte Wechselwirkung zwischen RNA-Polymerase und DNA ebenfalls eine erhöhte Genexpression bewirken.

Durch Klonierung des Gluconat-Oxidoreduktase-Gens sind Plasmide bzw. Vektoren erhältlich, die das Gen enthalten und zur Transformation eines 5-Ketogluconat bildenden Mikroorganismus geeignet sind. Die durch Transformation erhältlichen Zellen, bei denen es sich vorzugsweise um transformierte Zellen von Gluconobacter, insbesondere Gluconobacter oxydans, handelt, enthalten das Gen in replizierbarer Form, d.h. in zusätzlichen Kopien auf dem Chromosom, wobei die Genkopien durch homologe Rekombination an beliebigen Stellen des Genoms integriert werden, und/oder auf einem Plasmid bzw. Vektor.

Die Genisolierung, Klonierung und Transformation erfolgt nach den in der Molekularbiologie bekannten Methoden. Wird beispielsweise das Gen aus Gluconobacter, vorzugsweise aus Gluconobacter oxydans, isoliert, erfolgt die Isolierung basierend auf der Kenntnis der Aminosäuresequenz des entsprechenden Proteins. Zur Klonierung des Gens werden spezifische Gensonden eingesetzt. Das Gen kann nach Ligation mit einem für die Wirtszelle geeigneten Vektor- und Expressionssystem mit den gängigen Transfermethoden in die Wirtszelle eingebracht und dort zur Expression gebracht werden.

Wird als Wirtszelle beispielsweise Gluconobacter, vorzugsweise Gluconobacter oxydans, verwendet, bietet sich als Transformationsmethode insbesondere der bi- und triparentale konjugative Transfer an.

In einem mikrobiellen Herstellungsverfahren für 5-Ketogluconat wird durch Erhöhung der Genkopienzahl des Gluconat-Oxidoreduktase-Gens wie oben beschrieben die Genexpression eines 5-Ketogluconat bildenden Mikrorganismus, insbesondere Gluconobacter bzw. Gluconobacter oxydans, erhöht. Dabei können als Wirtszellen selbstverständlich auch solche Organismen dienen, die kein eigenes Gluconat:NADP⁺-5-Oxidoreduktase-Gen besitzen.

### Ausführungsbeispiel:

### Bestimmung der Enzymaktivität.

Bei der enzymatisch katalysierten Oxidation von Gluconat zu 5-Ketogluconat wurde NADP⁺ zu NADPH reduziert und photometrisch nachgewiesen (Okamoto, K. 1963. Enzymatic Studies on the Formation of 5-Ketogluconic Acid by Acetobacter suboxydans, J. Biochem. 53, 448-452).

### Produktbestimmung.

Die quantitative Analyse von Gluconat und 5-Ketogluconat erfolgte durch HPLC (R. Klasen et al. 1992. Incapability of Gluconobacter oxydans to Produce Tartaric Acid. Biotechnol. Bioeng. 40: 183-186).

### Reinigung der Gluconat: NADP⁺-5-Oxidoreduktase.

Anionenaustauscher: Zellfreier Extrakt von G. oxydans wurde auf einen Anionenaustauscher (DEAE-Tentakel, Merck Darmstadt; 5 15 cm) aufgetragen und gebunden. Das Enzym wurde mit einem steigenden NaCl-Gradienten (0 - 500 mM) bei 350 mM NaCl eluiert. Die aktiven Fraktionen wurden vereinigt und mit einer Ultrafiltrationseinheit (Amicon; 'cut off: 10 kDa) dialysiert.

Farbstoff-Affinitätschromatographie: Die dialysierte Enzymlösung wurde auf eine Farbstoff-Affinitätssäule (Blue Sepharose CL-6B™, 2,6 20 cm (T. Atkinson et al., 1981. Triazine-dye affinity chromatography. Biochem. Soc. Trans. 9: 10-13) aufgetragen und gebunden. Das Enzym wurde mit einem steigenden NaCl-Gradienten (0 - 500 mM) bei 300 mM NaCl eluiert. Die aktiven Fraktionen wurden vereinigt und mit einer Ultrafiltrationseinheit (Amincon; 'cut off: 10 kDa) gegen 25 mM Histidin/HCl Puffer pH 5,6 dialysiert.

Chromatofokussierung I: Die dialysierte Enzymlösung wurde auf eine mit 25 mM Histidin/HCl Puffer pH 5,6 äquilibrierte Mono-P™-Chromatographiesäule (Pharmacia, Freiburg; 0,5 20 cm) aufgetragen. Die Einstellung des pH-Gradienten und damit die Elution der Proteine erfolgte durch Applikation von 50 ml Polypuffer PB74 (Pharmacia, Freiburg) pH 4,0 auf die Säule.

Gelfiltration: Die vereinigten aktiven Fraktionen wurden auf eine Gelfiltrationssäule (Sephacryl-S100 HR, Pharmacia, Freiburg; 2,6 100 cm) appliziert. Die Elution erfolgte isokratisch. Die aktiven Fraktionen wurden vereinigt und mit einer Ultrafiltrationseinheit (Amicon; 'cut off: 10 kDa) gegen 2,5 mM Acetat/NaOH Puffer pH 4,8 dialysiert.

Chromatofokussierung II: Die Proteinlösung wurde auf eine mit 25 mM Acetat/NaOH Puffer pH 4,8 äquilibrierte Mono-P™-Chromatographiesäule (Pharmacia, Freiburg; 05, 20 cm) aufgetragen. Durch Applikation von 50 ml Polypuffer PB74 (Pharmacia, Freiburg) pH 4,0 wurde ein pH-Gradient auf der Säule eingestellt und das Protein eluiert. Nach Überprüfung der Reinheit durch SDS-PAGE wurde das gereinigte Protein aliquotiert, in flüssigem Stickstoff schockgefroren und bei -70°C gelagert.

### Aminoterminale Sequenzierung.

Für die aminoterminale Aminosäuresequenzierung wurde 100 µg gereinigtes Protein auf eine PVDF-Membran transferiert und mit einem Gasphasen-Sequenator (Appl. Biosystems, model 470A) untersucht (P. Edman et al., 1967. A Protein Sequenator. Eur. J. Biochem. 1: 80-91). Der Nachweis der abgespaltenen Aminosäurederivate (Phenylthiohydantoine) erfolgte durch HPLC.

### Herstellung der Gensonde.

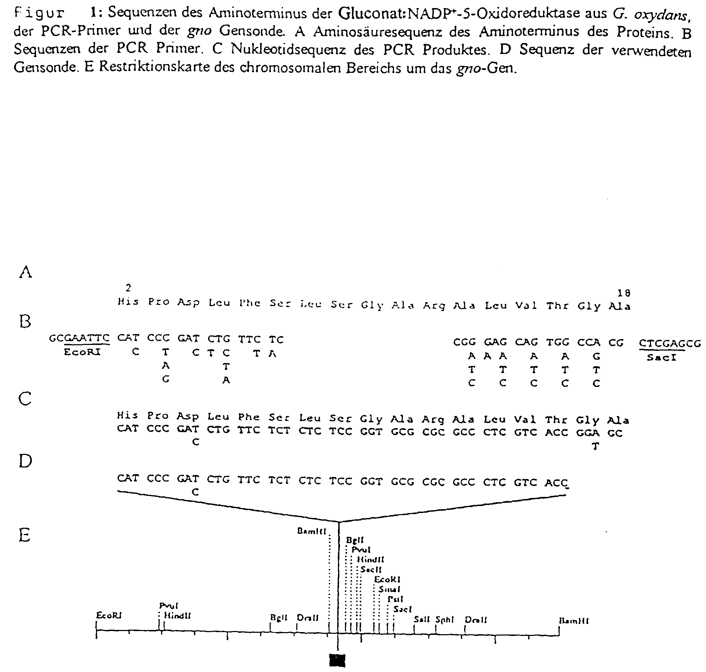

Molekularbiologische Arbeiten wurden, falls nicht gesondert aufgeführt, nach T. Maniatis et al. (1989, Molecular cloning: A laboratory manual 2nd edition. Cold Spring Harbour Laboratory, Cold Spring Harbour, N.Y.) durchgeführt. Durch Edman-Sequenzierung wurden die Aminosäuren 2 bis 18 der Gluconat: NADP⁺-5-Oxidoreduktase bestimmt (Figur 1A). Nach der Übersetzung der Proteinsequenz in die korrespondierende Nukleinsäureresequenz, unter Berücksichtigung der Degenerierung des genetischen Codes, ergab sich eine hoch degenerierte DNA-Sequenz. Zur Konstruktion einer gno-Gensonde wurden PCR-Primer entsprechend der Enden der ermittelten Peptidsequenz synthetisiert (Figur IB). An die 5'-Enden der Primer wurden Restriktionsschnittstellen für die Enzyme EcoRI und SacI angehängt, um bei der späteren Klonierung eine höhere Selektivität zu erreichen. Nach der Isolierung der Primer wurden diese für eine PCR-Reaktion mit gereinigter, chromsomaler DNA aus G. oxydans (Y. Takeda et al., 1991. Cloning and Sequencing of the Gene Encoding Cytochrome c-553 (CO) from Gluconobacter oxydans. J. Ferment. Bioeng. 72: 1-6) als Matrize eingesetzt (S.J. Gould et al., 1989. Use of the polymerase chain reaction for homology probing: Isolation of partial cDNA or genomic clones encoding the iron-sulfur protein of succinate debydrogenase from several species. Proc. Natl. Acad. Sci. USA 86; 1934-1938). Ein 66 bp-Fragment wurde nach präparativer, 4%iger Agarosegelelektrophorese isoliert und mit den Restriktionsendonukleasen EcoRI und SacI geschnitten. Nach Ligation des vorbereiteten Fragmentes mit entsprechend geschnittenem Plasmid pUC19 wurde es in E. coli kloniert. Das 66 bp PCR-Fragment wurde sequenziert, um eine nichtdegenerierte, den aminoterminalen Bereich des Proteins kodierende DNA-Sequenz zu ermitteln. Die DNA-Sequenz ließ sich der erhaltenen Peptidsequenz zuordnen (Figur 1C). Aus dem sequenzierten PCR-Fragment wurde ein Bereich ermittelt, der bei einer Länge von 45 bp dem GC-Gehalt von G. oxydans entsprach (60 - 65 %, J. de Ley et al., 1970. The status of the generic name Gluconobacter. Int. J. Syst. Bact. 20: 83-95). Diese Sequenz wurde als Muster für die Synthese einer gno-Gensonde verwendet (Figur 1D).

### Erstellung einer Restriktionskarte des gno-Gens.

Auf der Basis der Ergebnisse der PCR-Experimente wurde eine gno-Gensonde konstruiert und nach Digoxigeninmarkierung (G.G. Schmitz et al., 1991. Non-radioactive labeling of oligonucleotides in vitro with the hapten digoxigenin by tailing with Terminal Transferase. Anal. Biochem. 192: 222-231) in Southern-Blot Experimenten (E.M. Southern, 1975. Detection of specific sequences among DNA fragments seperated by gel electrophoresis. J. Mol. Biol. 8: 503-517) eingesetzt. Dazu wurde chromosomale DNA aus G. oxydans mit verschiedenen Kombinationen von Restriktionsendonukleasen geschnitten und nach Auftrennung der Restriktionsansätze durch Agarosegelelektrophorese auf eine Nylonmembran transferiert. Nach Hybridisierung mit der Gensonde zeigte sich pro Ansatz jeweils nur ein deutliches Signal. Durch Verrechnung der Größe der hybridisierenden Fragmente wurde eine Restriktionskarte ermittelt (Figur 1E).

### Klonierung des gno-Gens.

Nach Herstellung einer partiellen G. oxydans Genbank wurde ein 3,4 kb BamHI-Fragment, das das gesamte gno-Gen trug, durch Koloniehybridisierung kloniert (M. Grunstein et al., 1975. Colony hybridization: A method for the isolation of cloned DNA's that contain a specific gene. Proc. Natl. Acad. Sci. USA 72: 3961-3965).

### Sequenzierung des gno-Gens.

Die Sequenzierung des chromosomalen Bereichs um das gno-Gen (F. Sanger et al., 1977. DNA sequencing with chain termination inhibitors. Proc. Natl. Acad. Sci. USA 74: 5463-5467) ergab eine Nukleotidsequenz von 1510 bp mit einem offenen Leseraster von 771 bp, das für ein Polypeptid mit 257 Aminosäuren bei einem Molekulargewicht von 27.300 kodierte (Tabelle 1). Die Nukleotidsequenz hatte eine Ribosomenbindestelle 10 bp vor dem Startcodon (J. Shine et al., 1974. The 3'-terminal sequence of Escherichia coli 16S ribosomal RNA: Complementarity to nonsense triplets and ribosome binding sites. Proc. Natl. Acad. Sci. USA 71: 1342-1346) und eine terminatortypische Palindromstruktur 160 bp hinter dem Stopcodon (M. Rosenberg et al., 1979. Regulatory sequences involved in the promotion and termination of RNA transcription. Annu. Rev. Genet. 13: 319-353). Aus der abgeleiteten Aminosäuresequenz ergab sich die bei der Proteinsequenzierung erhaltene Peptidsequenz.

### Expression des gno-Gens in G. oxydans.

Als Vektor DNA für G. oxydans wurden Derivate des Plasmids RSF 1010 gewählt, da das Replikon auf diesem Plasmid die Replikation der DNA in einem breiten Spektrum Gram-negativer Bakterien erlaubte (U.B. Priefer et al., 1985. Extension of the host range of Escherichia coli vectors by incorporation of RSF 1010 replication and mobilization functions. J. Bacteriol. 163: 324-330). Als Basis für weitere Untersuchungen zum Gentransfer in G. oxydans wurde das Plasmid pRS201P_{R} verwendet (R. Schröder et al., 1991. Expression of the core antigen of hepatitis B virus (HBV) in Acetobacter methanolicus using broad-host-range vectors. Appl. Microbiol. Biotechnol. 35: 631-637). Der Plasmidtransfer erfolgte durch Konjugation (C. Condon et al., 1991. Conjugation and heterologous gene expression in Gluconobacter oxydans ssp. suboxydans. FEMS Microbiol. Lett. 80: 173-178). Das Plasmid pRS201P_{R} enthielt neben dem Promotor auch das Gen für ein temperatursensitives Derivat des cI-Repressors (cI857), der den Promotor regulierte. Um den Promotor zu deregulieren, wurde ein Teil des cI857-Repressors vom Plasmid pRS201P_{R} eliminiert (pRS201P). Ein für die Gluconat: NADP⁺-5-Oxidoreduktase kodierendes, 1,25 kb BamHI/SalI-Fragment wurde mit entsprechend restringiertem pRS201P_{R} und pRS201P ligiert. Die Plasmide pRS201P_{R}-gno und pRS201P-gno wurden durch Konjugation auf G. oxydans übertragen. Die Aktivitätsbestimmung zeigte eine 6,5 bis 85-fache Erhöhung der Gluconat: NADP⁺-5-Oxidoreduktase-Aktivität in den rekombinanten G. oxydans-Stämmen (Tabelle 2).

### 5-Ketogluconatproduktivität der rekombinanten Stämme.

Zur Bestimmung der 5-Ketogluconatproduktivität der rekombinanten G. oxydans-Stämme wurde die Akkumulation von 5-Ketogluconat im Fermentationsmedium untersucht (U. Kotera et al., 1972. Isolation and chemical structure of new oxidation product of 5-ketogluconic acid, and a hypothetical pathway from glucose to tartaric acid through this new compound. Agric. Biol. Chem. 36: 1315-1325). Täglich wurden Proben für die Metabolitbestimmung entnommen und durch HPLC-Messungen quantitativ bestimmt. Es zeigte sich eine Erhöhung der 5-Ketogluconatbildung bei Überexpression des gno-Gens (G. oxydans pRS201P-gno) im Vergleich zu den Stämmen mit der Gluconat: NADP⁺-5-Oxidoreduktase-Aktivität des Wildtyps (G. oxydans wt, G. oxydans pRS201P) um 11 % (Tabelle 3).

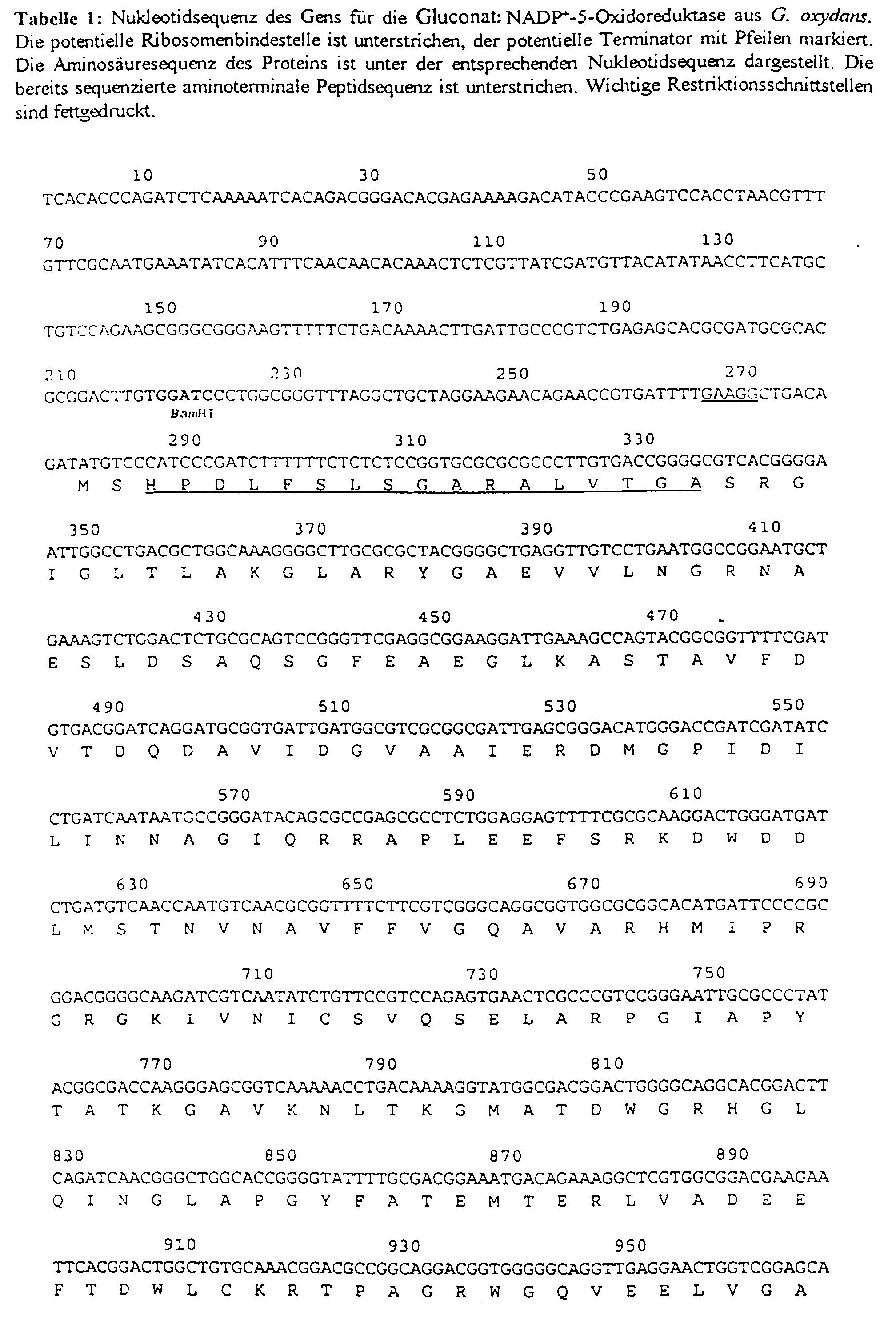

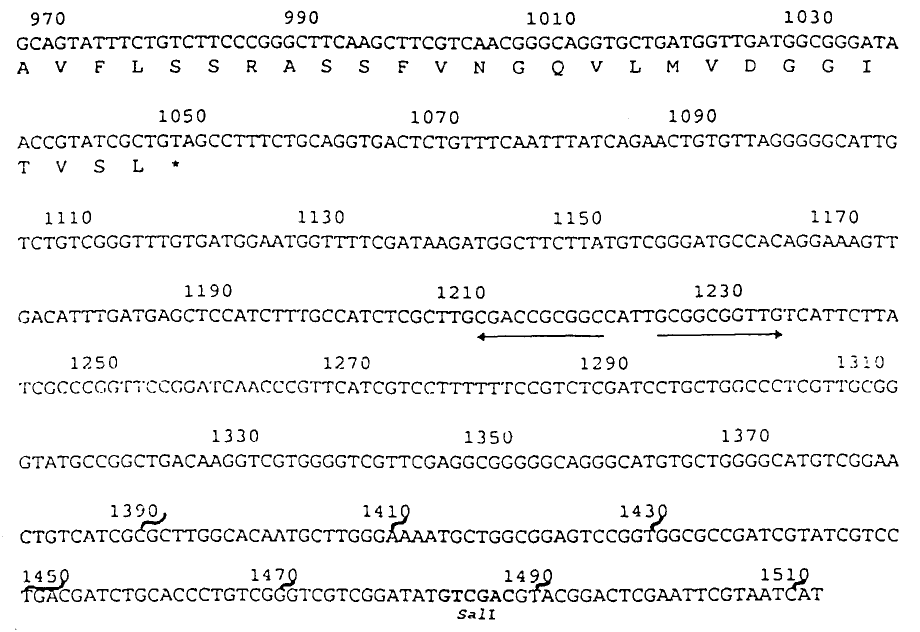

**Tabelle 2:**

| Expression des *gno*-Gens in *G. oxydans* DSM 3503 unter Kontrolle des λ Promotors in den Plasmiden pRS201P_{R}-*gno* und pRS201P-*gno* und die resultierende Gluconat: NADP⁺-5-Oxidoreduktase-Aktivität. Die Enzymaktivität wurde nach 20 h Inkubation bei 30°C bestimmt. | |
|---|---|
| Stamm | Enzymaktivität [U/mg] |
| *G*. *oxydans* | 0,08 |
| *G*. *oxydans* pRS201P_{R} | 0,08 |
| *G*. *oxydans* pRS201P | 0,08 |
| *G*. *oxydans* pRS201P_{R}-*gno* | 0,52 |
| *G*. *oxydans* pRS201P-*gno* | 6,80 |

**Tabelle 3:**

| Umsetzung von Glukose zu 5-Ketogluconat durch rekombinante *G*. *oxydans* Stämme. Die Inkubation erfolgte in Mineralsalzmedium mit 10 % (w/v) Glukose. | | |
|---|---|---|
| Stamm | Zeit [d] | 5-Ketoglukonat [mM] |
| *G*. *oxydans* wt | 1 | 15 |
| | 2 | 56 |
| | 3 | 71 |
| | | |
| *G*. *oxydans* pRS201P | 1 | 12 |
| | 2 | 58 |
| | 3 | 69 |
| | | |
| *G*. *oxydans* pRS201P-*gno* | 1 | 19 |
| | 2 | 66 |
| | 3 | 79 |

## Patentansprüche

1. Gluconat: NADP⁺-5-Oxidoreduktase-Gen mit einer für die in Tabelle 1 angegebene Aminosäuresequenz oder deren Allelvariationen kodierenden Nukleotidsequenz.

2. Gluconat: NADP⁺-5-Oxidoreduktase-Gen nach Anspruch 1 oder ein funktionelles Derivat davon, das durch eine oder mehrere Deletionen, eine oder mehrere Insertionen oder eine oder mehrere Substitutionen in der Sequenz gemäß Tabelle 1 erhältlich ist, und wobei das Genprodukt Gluconat: NADP⁺-5-Oxidoreduktase-Aktivität aufweist.

3. Gluconat: NADP⁺-5-Oxidoreduktase-Gen nach Anspruch 1 oder 2 mit einer diesem zugeordneten regulatorischen Gensequenz.

4. Genkonstrukt, enthaltend ein Gluconat:NADP⁺-5-Oxidoreduktase-Gen nach einem der Ansprüche 1 bis 3.

5. Vektor, enthaltend ein Gluconat:NADP⁺-5-Oxidoreduktase-Gen nach einem der Ansprüche 1 bis 3 oder ein Genkonstrukt nach Anspruch 4.

6. Transformierte Zelle, enthaltend in replizierbarer Form ein Gluconat:NADP⁺-5-Oxidoreduktase-Gen nach einem der Ansprüche 1 bis 3 oder ein Genkonstrukt nach Anspruch 4.

7. Transformierte Zelle nach Anspruch 6, enthaltend einen Vektor nach Anspruch 5.

8. Transformierte Zelle nach Anspruch 6 oder 7, **dadurch gekennzeichnet**, daß sie Gluconobacter, vorzugsweise Gluconobacter oxydans, ist.

9. Verfahren zur mikrobiellen Herstellung von 5-Ketogluconat, bei dem die Gluconat: NADP⁺-Oxidoreduktase-Genexpression eines 5-Ketogluconat bildenden Mikroorganismus durch Erhöhen der Genkopienzahl des Gluconat:NADP⁺-5-Oxidoreduktase-Genes nach einem der Ansprüche 1 bis 3 erhöht wird.

10. Verfahren nach Anspruch 9, **dadurch gekennzeichnet**, daß der 5-Ketogluconat bildende Mikroorganismus ein Gluconobacter, vorzugsweise Gluconobacter oxydans ist.

11. Verfahren nach einem der Ansprüche 9 oder 10, **dadurch gekennzeichnet**, daß das Gluconat:NADP⁺-5-Oxidoreduktase-Gen aus Gluconobacter, vorzugsweise Gluconobacter oxydans, isoliert wird.

## Claims

1. Gluconate: NADP⁺-5-oxidoreductase gene which possesses a nucleotide sequence which encodes the amino acid sequence given in Table 1 or its allele variations.

2. Gluconate: NADP⁺-5-oxidoreductase gene according to Claim 1, or a functional derivative thereof, which can be obtained by means of one or more deletions, one or more insertions or one or more substitutions in the sequence depicted in Table 1, and with the gene product exhibiting gluconate: NADP⁺-5-oxidoreductase activity.

3. Gluconate: NADP⁺-5-oxidoreductase gene according to Claim 1 or 2 which possesses a regulatory gene sequence which is assigned to this gene.

4. Gene construct which contains a gluconate: NADP⁺-5-oxidoreductase gene according to one of Claims 1 to 3.

5. Vector which contains a gluconate: NADP⁺-5-oxidoreductase gene according to one of Claims 1 to 3 or a gene construct according to Claim 4.

6. Transformed cell which contains, in replicatable form, a gluconate: NADP⁺-5-oxidoreductase gene according to one of Claims 1 to 3 or a gene construct according to Claim 4.

7. Transformed cell according to Claim 6 which contains a vector according to Claim 5.

8. Transformed cell according to Claim 6 or 7, characterized in that it is Gluconobacter, preferably Gluconobacter oxydans.

9. Process for microbially preparing 5-ketogluconate in which the gluconate: NADP⁺-oxidoreductase gene expression of a 5-ketogluconate-forming microorganism is increased by increasing the gene copy number of the gluconate: NADP⁺-5-oxidoreductase gene according to one of Claims 1 to 3.

10. Process according to Claim 9, characterized in that the 5-ketogluconate-forming microorganism is a Gluconobacter, preferably Gluconobacter oxydans.

11. Process according to Claim 9 or 10, characterized in that the gluconate: NADP⁺-5-oxidoreductase gene is isolated from Gluconobacter, preferably Gluconobacter oxydans.

## Revendications

1. Gène de gluconate: NADP⁺-5-oxydoréductase possédant une séquence d'acides aminés indiquée dans le tableau 1 ou sa séquence nucléotidique codant des variations alléliques.

2. Gène de gluconate: NADP⁺-5-oxydoréductase selon la revendication 1 ou un de ses dérivés fonctionnels, que l'on obtient via une ou plusieurs délétions, via une ou plusieurs insertions ou via une ou plusieurs substitutions dans la séquence selon le tableau 1, et dans lequel le produit génique présente une activité de gluconate: NADP⁺-5-oxydoréductase.

3. Gène de gluconate: NADP⁺-5-oxydoréductase selon la revendication 1 ou 2, comprenant une séquence génique régulatrice qui lui est attribuée.

4. Constituant du gène contenant un gène de gluconate: NADP⁺-5-oxydoréductase selon l'une quelconque des revendications 1 à 3.

5. Vecteur contenant un gène de gluconate: NADP⁺-5-oxydoréductase selon l'une quelconque des revendications 1 à 3 ou un constituant du gène selon la revendication 4.

6. Cellule transformée contenant, sous une forme réplicable, un gène de gluconate: NADP⁺-5-oxydoréductase selon l'une quelconque des revendications 1 à 3 ou un constituant du gène selon la revendication 4.

7. Cellule transformée selon la revendication 6, contenant un vecteur selon la revendication 5.

8. Cellule transformée selon la revendication 6 ou 7, caractérisée en ce qu'il s'agit d'un Gluconobacter, de préférence de Gluconobacter oxydans.

9. Procédé pour la préparation microbienne du 5-cétogluconate, dans lequel on augmente l'expression du gène de gluconate: NADP⁺-5-oxydoréductase d'un micro-organisme formant du 5-cétogluconate en augmentant le nombre de copies géniques du gène de gluconate: NADP⁺-5-oxydoréductase selon l'une quelconque des revendications 1 à 3.

10. Procédé selon la revendication 9, caractérisé en ce que le micro-organisme formant le 5-cétogluconate est un Gluconobacter, de préférence Gluconobacter oxydans.

11. Procédé selon la revendication 9 ou 10, caractérisé en ce qu'on isole le gène de gluconate: NADP⁺-5-oxydoréductase à partir d'un Gluconobacter, de préférence à partir de Gluconobacter oxydans.
